# EUROPEAN PATENT APPLICATION

(11) **EP 4 529 947 A1**
(43) Date of publication of application: **02.04.2025**
(21) Application number: 23201051.2
(22) Date of filing: 29.09.2023
(51) Int. Cl.: A61N 5/06

(54) **SYSTEM FOR CONTROLLING A LIGHT SOURCE**

(71) Applicant: Skinuvita GmbH, 28195 Bremen (DE)
(72) Inventor: Pelzetter, Jens, 28217 Bremen (DE); Elsner, Jan, 28201 Bremen (DE)
(74) Representative: Eisenführ Speiser

(57) **Abstract**

The invention relates to a system 1 for controlling a light source 2. A therapy planner 3 allows a user 4 to input therapy parameters usable for generating a light therapy plan for a patient 5, which defines several sessions of a light therapy to be applied to the skin of the patient and comprises dose data indicative of the radiation dose to be applied during a respective session and time data indicative of the time at which the respective session is to be carried out. A server 6 calculates control data for the several sessions based on the dose data of the light therapy plan, wherein the control data define an operation of the light source 2 during the respective session. A patient device 7 receives the control data from the server 6 and transfers the control data to a connector 8 which controls the light source in accordance with the received control data.

## Description

### FIELD OF THE INVENTION

The invention relates to a system, method and computer program for controlling a light source. The invention further relates to a therapy planner, a server, a patient device and a connector of the system for controlling the light source. The invention also relates to methods to be carried out by the entire system and by the therapy planner, the server, the patient device and the connector. Moreover, the invention relates to computer programs to be carried out by the entire system and by the therapy planner, the server, the patient device and the connector.

### BACKGROUND OF THE INVENTION

Treating skin by light generally requires that a patient with skin problems visits a physician in a medical office or a hospital several times a week. This takes a lot of time for the patient, wherein often it is difficult to, for instance, plan the regular work such that the physician can be visited and hence the skin treatment can be carried out at the site of the physician. For some patients it might not even be possible to visit the physician several times a week. Also for this reason, patients often prefer treating the skin problems by medication which might have side effects because the medication generally is not completely focused on the skin treatment, but also adversely influences other parts of the patient.

### SUMMARY OF THE INVENTION

It is hence an object of the present invention to provide a system, a method and a computer program for controlling a light source, which allows the patient to carry out the treatment of the skin by using light at the patient's site, for instance, at home, wherein still the treatment can be carried out reliably with a good healing effect. It is a further object of the present invention to provide a therapy planner, a server, a subject device and a connector to be used by the system for controlling the light source and corresponding methods and computer programs.

In a first aspect of the invention a system for controlling a light source is presented, wherein the system comprises:
- a therapy planner configured to allow a user to input therapy parameters that are usable for generating a light therapy plan for a patient, wherein the light therapy plan defines several sessions of a light therapy to be applied to the skin of the patient and comprises dose data indicative of the radiation dose to be applied during a respective session and time data indicative of the time at which the respective session is to be carried out,
- a server configured to calculate the light therapy plan based on the input therapy parameters and to calculate control data for the several sessions based on the dose data of the light therapy plan, wherein the control data define an operation of the light source during the respective session,
- a patient device configured to receive the control data from the server and to transfer the control data to a connector, and
- the connector configured to connect the patient device with the light source, wherein the connector is configured to control the light source in accordance with the received control data.

Thus, a user like a physician can input therapy parameters, which are usable for generating the light therapy plan for a patient, at the site of the physician, for instance, in a medical office or a hospital, while the light source can nevertheless be accurately controlled at the site of the patient by using the server, the patient device and the connector. It is hence possible to treat the skin of the patient by using light of the light source at the site of the patient, without requiring the patient to visit the physician in his medical office or in a hospital several times a week.

The light source is preferentially an ultraviolet (UV) light source and the therapy planner preferentially is a computer at the site of the physician, wherein a software is running on the computer which provides the therapy planning functionality to the physician.

The server can be located anywhere. It even can be a cloud server or another type of server, it is just required that the server has a data connection to the therapy planner and to the patient device.

The patient device is a patient device to be used at the site of the patient, for instance, at the home of the patient. It can be a computer device, especially a mobile computer device like a smartphone or a tablet. A software can run on the computer device for providing the functionality of the patient device as described herein.

The connector can be a hardware component that is configured to wirelessly connect to the patient device, for instance, to a smartphone of the patient, and to provide a wired or wireless connection to the light source. Also the data connection between the patient device and the connector can be a wired connection.

The connector can be integrated in or with the light source, or it can be a separate device which is connected to the light source via, for instance, a cable.

Preferentially, the server comprises light source data indicative of light source characteristics, wherein the server is configured to calculate the control data further based on the light source characteristics. The light source characteristics can include information about the spectral distribution, particular an UV band, of the light and/or the light intensity providable by the light source at least one distance between the light source and the skin of the patient. Further, the server can comprise patient data, wherein the server can be configured to calculate the therapy plan also based on the patient data. The patient data can include information about at least one of a) the skin type of the patient, b) the medication of the patient, c) diagnoses of the patient and d) feedback data from the patient about effects of the light therapy. The feedback data can include redness of the treated skin area and/or a surrounding skin area, pain in the treated skin area, et cetera. The control data preferentially includes a time period of applying the radiation during the respective session. The server can be further configured to determine guidance data for guiding the patient before and/or during a session and to provide the guidance data to the patient device. The guidance data can include at least one of a) a distance between the light source and the skin to be used during the respective session, b) guidance regarding clothing of the patient during the respective session and c) whether to wear safety glasses. These features allow for a very reliable control of the light source at the site of the patient, although a healthcare professional like a physician is not present. Moreover, the light source itself can be relatively simple. It can even be a known generally used light source. In a preferred embodiment, the light source just needs to have the ability to change its illumination duration, in order to be usable by the described system.

In a preferred embodiment a bi-directional communication i) between the server and the therapy planner and/or ii) between the server and the patient device is initiated only by the therapy planner and the patient device, respectively, and not by the server. Thus, the server bi-directionally communicates with the therapy planner and/or with the patient device only if such a communication is requested by the therapy planner and the patient device, respectively. In particular, the bi-directional communication i) between the server and the therapy planner and/or ii) between the server and the patient device is present only, if such a bi-directional communication is needed really and therefore initiated by the therapy planner or the patient device, respectively. Therefore, no permanent bi-directional data connection is required i) between the server and the therapy planner and/or ii) between the server and the patient device. For example, only if the therapy planner or the patient device needs data from the server, a bi-directional data connection i) between the server and the therapy planner or ii) between the server and the patient device is established and then used for the respective bi-directional communication. Since a permanent bi-directional data connection is not required by the system, a breakdown or interruption of the bi-directional data connection has no or substantially no effect on the operability of the system. The system therefore is very tolerant regarding data connection failures.

In an example, the server is configured to i) check whether a next session is available based on a current time and the time data of the different sessions, if the patient device requests control data for the next session, and ii) provide the control data of the next session to the patient device, if the check revealed that a next session is available. Moreover, the server can be configured to inform the patient device when a next session is to be carried out based on the time data of the sessions as defined by the therapy plan. In particular, the server provides control data for controlling the light source to the patient device only when the corresponding session should now or shortly be carried out. The server hence preferentially does not send control data for several sessions in advance. If a current session is to be carried out, the patient device only comprises the control data of the current session and optionally, if not removed by the patient device, control data of previous sessions, but not control data of future sessions. Moreover, preferentially the server determines, i.e. calculates, the control data of a next session, particularly the illumination time period of the next session, only, after the patient device has requested the next session. Preferentially, before having received such a request from the patent device, the server does not calculate the illumination time period of the next session. This reduces the likelihood that wrong control data are used and thus that the light source is wrongly controlled during a session, thereby further increasing the reliability of the control of the light source, although no healthcare professional like a physician or another expert is present at the site of the patient, for instance, at the patient's home.

The therapy planner can be configured to allow a user to perform at least one of a) monitoring feedback about the light therapy defined by the therapy plan, b) modifying the light therapy plan, c) releasing the light therapy plan, d) pausing the light therapy plan and e) cancelling the light therapy plan. Moreover, in an example, the patient device is configured to a) provide the patient with information about a current state of the light therapy, b) allow the patient to input feedback data about effects of the light therapy, and c) provide guidance of the patient during the respective session. In particular, the patient device can be configured to ask the server whether it is possible to provide feedback and to allow the patient to input feedback, if the server has informed the patient device that feedback is possible. Thus, in an embodiment, the patient device is able to allow the user to input feedback, only if the server agrees with this. The server can comprise feedback rules defining under which conditions the user is allowed to input feedback and to inform the patient device whether feedback is possible or not based on these rules. For instance, these rules can define that the feedback can be provided only within a time interval relative to the last session. Preferentially, this time interval ends before the start time of the planned next session. If it is assumed that the sessions should be carried out at the same time of the day, the time interval for providing feedback is defined such that it ends before 24 hours after the start time of the previous session, in order to avoid a "slow drift" of the starting times of the sessions from day to day. For instance, the time interval for providing feedback can end 22 hours or 23 hours after the start time of the previous session. The width of the time interval is chosen such that the user has sufficient time for providing the feedback. The width can be, for instance, one hour, 30 minutes or 15 minutes.

In an example the server is configured to inform the patient device about the treated skin areas, wherein the patient device is configured to ask the patient for feedback regarding the treated skin areas. The feedback therefore can be more focused. In particular, it can be asked exactly for the feedback which is required for improving the therapy plan. This in turn can further increase the quality of the skin treatment carried out at the site of the patient.

In an example the patient device includes a camera and is configured to ask the patient to make a photo of the treated skin area and to send the photo to the server as feedback. The server can be configured to analyze the photo, for example by artificial intelligence, and to adapt the therapy plan based on the result of the analyses. This can increase the objectivity and thereby the quality of the therapy plan, because the feedback and therefore the feedback-based adaptation of the therapy plan is not or not only based on the subjective impression of the patient, but on the photo of the treated skin area and the analysis of the photo. This can further increase the reliability and hence quality of the treatment of the skin at the site of the patient, although a healthcare professional is not present at the site of the patient.

In a further aspect of the present invention, a therapy planner for the system for controlling a light source is presented, wherein the therapy planner is configured to allow a user input therapy parameters that are usable for generating a light therapy plan for a patient, wherein the light therapy plan defines several sessions of a light therapy to be applied to the skin of a patient and comprises dose data indicative of the radiation dose to be applied during a respective session and time data indicative of the time at which the respective session is to be carried out.

Moreover, in an aspect of the present invention, a server for the system for controlling a light source is presented, wherein the server is configured to calculate the light therapy plan based on the input therapy parameters and to calculate control data for the several sessions based on the dose data of the light therapy plan, wherein the control data define an operation of the light source during the respective session.

In a further aspect of the present invention, a patient device for the system for controlling a light source is presented, wherein the patient device is configured to receive the control data from the server and to transfer the control data to a connector.

Furthermore, in an aspect of the present invention, a connector for the system for controlling a light source is presented, wherein the connector is configured to connect the patient device with the light source, wherein the connector is configured to control the light source in accordance with the received control data.

In an aspect of the present invention, a method for controlling a light source is presented, wherein the method comprises
- allowing a user to input therapy parameters that are usable for generating a light therapy plan for a patient by a therapy planner, wherein the light therapy plan defines several sessions of a light therapy to be applied to the skin of the patient and comprises dose data indicative of the radiation dose to be applied during a respective session and time data indicative of the time at which the respective session is to be carried out,
- calculating the light therapy plan based on the input therapy parameters and calculating control data for the several sessions based on the dose data of the light therapy plan by a server, wherein the control data define an operation of the light source during the respective session,
- receiving the control data from the server and transferring the control data to a connector by a patient device,
- controlling the light source in accordance with the received control data by the connector.

In a further aspect of the present invention, a therapy planning method to be carried out by the therapy planner of the system for controlling a light source is presented, wherein the therapy planner allows a user to input therapy parameters that are usable for generating a light therapy plan for a patient, wherein the light therapy plan defines several sessions of a light therapy to be applied to the skin of a patient and comprises dose data indicative of the radiation dose to be applied during a respective session and time data indicative of the time at which the respective session is to be carried out.

Moreover, in an aspect of the present invention, a method to be carried out by the server of the system for controlling a light source is presented, wherein the server calculates the light therapy plan based on the input therapy parameters and calculates control data for the several sessions based on the dose data of the light therapy plan, wherein the control data define an operation of the light source during the respective session.

In an aspect of the present invention, a method to be carried out by the patient device of the system for controlling a light source is presented, wherein the patient device receives the control data from the server and transfers the control data to a connector.

In a further aspect of the present invention a method to be carried out by the connector of the system for controlling a light source is presented, wherein the connector controls the light source in accordance with the received control data.

Moreover, in an aspect of the present invention, a computer program for controlling the light source is presented, wherein the computer program comprises program code means for causing the system for controlling the light source to carry out the steps of claim 12.

In an aspect of the present invention, a computer program for controlling the therapy planner of the system for controlling a light source is presented, wherein the computer program comprises program code means for causing the therapy planner to carry out the therapy planning method.

Furthermore, in an aspect of the present invention, a computer program for controlling the server of the system for controlling a light source is presented, wherein the computer program comprises program code means for causing the server to carry out the method to be carried out by a server as described above.

In a further aspect of the present invention, a computer program for controlling the patient device of the system for controlling a light source is presented, wherein the computer program comprises program code means for causing the patient device to carry out the method to be carried out by a patient device described above.

Moreover, in an aspect of the present invention, a computer program for controlling the connector of the system for controlling a light source is presented, wherein the computer program comprises program code means for causing the connector to carry out the method as defined by claim 13.

It shall be understood that the system for controlling a light source of claim 1, the therapy planner, the connector of claim 11, the method for controlling a light source of claim 12, the therapy planning method, the method to be carried out by the server, the method to be carried out by the patient device, the method of claim 13, the computer program of claim 14, the computer program for controlling the therapy planner, the computer program for controller the server, the computer program for controlling the patient device and the computer program of claim 15 have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE EMBODIMENTS

In the following the drawings:
- Fig. 1: shows schematically and exemplarily a system for controlling a light source,
- Fig. 2: shows a flow chart exemplarily illustrating an embodiment of a method for controlling a light source,
- Fig. 3: shows schematically and exemplarily components of the system for controlling a light source,
- Figs. 4a, 4b, 4c: exemplarily show a flow chart illustrating details of the method for controlling a light source,
- Fig. 5: shows a UML sequence diagram exemplarily illustrating an execution of a session of a therapy plan,

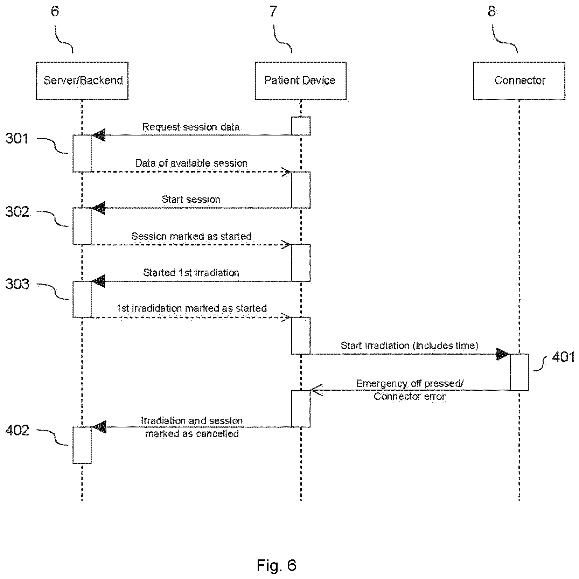

- Fig. 6: shows a further UML sequence diagram exemplarily illustrating a cancellation of an irradiation of a session of a therapy plan,
- Fig. 7: shows a UML sequence diagram exemplarily illustrating a communication during a waiting period and
- Fig. 8: shows a further UML sequence diagram exemplarily illustrating providing feedback and reviewing and updating a therapy plan.

### DETAILED DESCRIPTION OF EMBODIMENTS

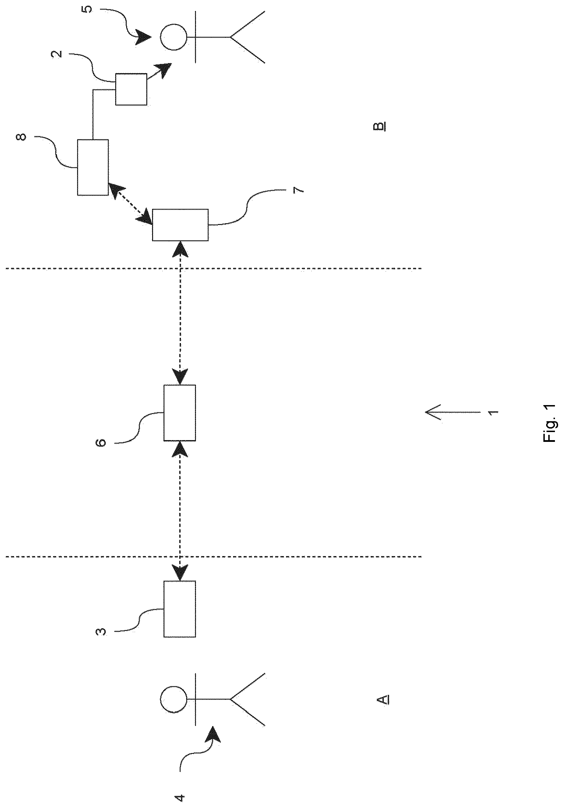

Fig. 1 shows schematically and exemplarily a system 1 for controlling a light source 2. The system 1 comprises a therapy planner 3 configured to allow a healthcare professional to input therapy parameters that are usable for generating a light therapy plan for a patient 5, wherein the light therapy plan defines several sessions of a light therapy to be applied to the skin of the patient 5. The therapy plan comprises dose data indicative of the irradiation dose to be applied during a respective session and time data indicative of the time at which the respective session is to be carried out. The therapy planner 3 is located at the site A of the healthcare professional, for instance, in a medical office of the healthcare professional or a hospital, while the patient 5 is at a patient's site B being, for instance, the patient's home.

The system 1 further comprises a server 6 configured to calculate the light therapy plan based on the input therapy parameters and to calculate control data for the several sessions based on the dose data of the light therapy plan, wherein the control data define an operation of the light source 2 during the respective session. The server 6 can be located anywhere. In particular, generally it will not be located at the site A of the healthcare professional 4 and also not at the site B of the patient 5.

The server 6 has a data connection to the therapy planner 3 and also to a patient device 7. The data connection between the server 6 and the therapy planner 3 and the data connection between the server 6 and the patient device 7 are preferentially provided via the Internet.

The system 1 further comprises a connector 8 configured to connect the patient device 7 with the light source 2, wherein the connector 8 is configured to control the light source 2 in accordance with the received control data. The data connection between the patient device 7 and the connector 8 can be a wired or wireless data connection and preferentially is a wireless data connection like Bluetooth. The data connection between the connector 8 and the light source 2 also can be a wired or a wireless data connection and preferentially is a wired data connection.

The server 6 comprises light source data indicative of light source characteristics and patient data, wherein the server is configured to calculate the control data further based on the light source characteristics. The light source characteristics preferentially include the spectral distribution of the light, i.e., for instance, the spectral location of an UV band of the light, and the light intensity provided by the light of the light source 2 at one or at different distances between the light source 2 and the skin of the patient 5. The server 6 can be configured to determine the control data such that they include a time period of applying the irradiation during the respective session and the server 6 optionally can also be configured to determine guidance data for guiding the patient 5 before and/or during a session and to provide the guidance data to the patient device 7. The guidance data, which can also be regarded as being guidance information and which includes the information for guiding the patient 2, can include a distance between the light source 2 and the skin of the patient 5 to be used during the respective session. It can also include a guidance regarding clothing of the patient 5 during the respective session and whether to wear safety glasses.

The patient data can include information about, for instance, the skin type of the patient 5, a medication of the patient 5, previous diagnoses of the patient 5 and feedback data from the patient 5 about effects of the light therapy. The feedback data can include, for instance, a degree of redness of the treated skin area and/or of a surrounding skin area, pain in the treated skin area, et cetera. The server 6 can be configured to calculate the light therapy plan further based on the patient data.

The server 6 is preferentially configured such that the bi-directional communication between the server 6 and the therapy planner 3 and the bi-directional communication between the server 6 and the patient device 7 is not initiated by the server 6, but by the therapy planner 3 and the patient device 7, respectively. For instance, the patient device 7 can request control data for a next session, wherein in response to this request the server 6 can check whether a next session is available based on a current time and the time data of the different sessions. If this check reveals that a next session is available, the server 6 provides the control data of the next session to the patient device 7. If a next session is not available, the server 6 can inform the patient device 7 when the next session to be carried out will be available based on the time data of the sessions defined by the therapy plan.

The therapy planner 3 is configured to allow the healthcare professional 4 to perform at least one of monitoring feedback about the light therapy, modifying the light therapy plan, releasing the light therapy plan, pausing the light therapy plan and cancelling the light therapy plan. The patient device 7 is configured to provide the patient 5 with information about the current state of the light therapy, to allow the patient 5 to input feedback data about effects of the light therapy and to provide guidance of the patient 5 during the respective session or before the respective session based on the guidance data received from the server 6.

The patient device 7 can be configured to ask the server 6 whether it is possible, given feedback rules of the server 6, to provide feedback and to allow the patient 5 to input feedback, if the server 6 has informed the patient device 7 that feedback is possible. The server 6 can also inform the patient device 7 about the treated skin areas, i.e. about the locations of the treated skin areas, wherein then the patient device 7 can ask the patient 5 for feedback specifically regarding the treated skin areas.

In an embodiment the patient device 7 includes a camera and is configured to ask the patient 5 to make a photo of the treated skin area and to send the photo to the server 6 as feedback. The servers can then be configured to analyze the photo, particularly by artificial intelligence, and to adapt the therapy plan based on the result of the analysis. The artificial intelligence can be trained to recognize, for instance, erythema on a photo of the skin. The artificial intelligence can particularly be trained to classify erythema according to the degree or level of redness. For the training of the artificial intelligence, a training data set is used, which comprises a number of photos of the skin of different persons as training photos and known degrees of redness, which could be regarded as being training labelings. The training labelings are determined by an expert like a physician. The artificial intelligence preferentially is a neural network module, particularly a convolutional neural network (CNN). The artificial intelligence can also be trained to output a score being indicative for a degree of severity of a skin defect. For instance, the artificial intelligence can be trained to output a Psoriasis Area and Severity Index (PASI) as the score or to output an Eczema Area and Severity Index (EASI) as the score. Also in this case a training data set is used, wherein the training data set includes training photos of the skin of different persons and known scores, which could also be regarded as being training scores or training labelings. The artificial intelligence is trained such that deviations between a) labelings, i.e. in the above examples degrees of redness or scores, output by the artificial intelligence, in response to being provided with the training photos as input, and b) the corresponding training labelings are minimized. Also in the case of the scores, the artificial intelligence is preferentially a neural network module, particularly a CNN module.

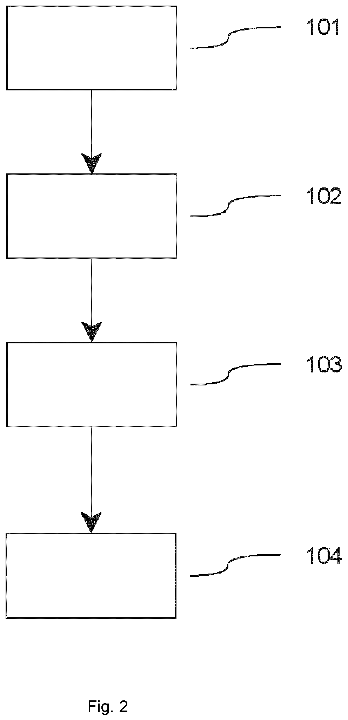

Fig. 2 shows exemplarily a flowchart illustrating an embodiment of a method for controlling a light source. In step 101, a healthcare professional 4 is allowed to input therapy parameters that are usable for generating a light therapy plan for a patient 5 by using the therapy planner 3. The light therapy plan defines several sessions of a light therapy to be applied to the skin of the patient 5 and comprises dose data indicative of the irradiation doses to be applied during a respective session and time data indicative of the time at which the respective session is to be carried out. In step 102, the light therapy plan is calculated based on the input therapy parameters and also the control data are calculated for the several sessions based on the dose data of the light therapy plan by the server 6. The control data define the operation of the light source 2 during the respective session. For instance, they define the duration of illuminating the skin with UV light during a respective session. In step 103, the patient device 7 receives the control data from the server 6 and transfers the received control data to the connector 8. In step 104, the light source 2 is controlled by the connector 8 in accordance with the received control data.

Step 101 could be regarded as being a step of a therapy planning method and the calculation of the light therapy plan and of the control data and the sending of the control data to the patient device 7 in step 102 can be regarded as being steps of a method to be carried out by the server 6. The receiving of the control data from the server 6 and the transferring of the control data to the connector 8 by the patient device 7 as defined in claim 103 can be regarded as being steps of a method to be carried out by the patient device 7. The receiving of the control data by the connector and the control of the light source 2 in accordance with the received control data performed by the connector 8 can be regarded as being steps of a method to be carried out by the connector 8.

The system 1 provides a telehealth solution for treating chronic skin diseases using UV light. This phototherapy is very effective and has minimal side effects. The skin diseases that can be cured using the irradiation with UV light are photoresponsive diseases like psoriasis, atopic dermatitis and atopic eczema, vitiligo, early-stage Mycosis fungoides, large plaque parapsoriasis, prophylaxis of polymorphic light dermatosis, pruritus, prurigo, et cetera.

The therapy planner 3 could also be regarded as being a therapy manager or therapy manager front end of the system 1. The therapy planner 3 can be implemented as a web application or as another software running on a computer at the side A of the healthcare professional 4.

The therapy planner 3 can be configured to be used by the healthcare professional 4 to input therapy parameters and to thereby, with the help of the servers, create therapy plans. The therapy planner 3 also can be configured to monitor the therapy plans for patients. The therapy planner 3 can also be configured to allow the healthcare professional 4 to monitor the feedback from the patient 5 and to adapt the therapy plan, if regarded as being necessary, by the healthcare professional 4.

The server 6 can be configured to automatically stop the therapy plan based on the feedback data received from the patient via the patient device 7 by using stopping rules defined on the server 6. The stopping rules can define, for instance, that the therapy plan should be stopped automatically, if the feedback data indicate a certain degree of redness of the treated skin area and/or of skin areas surrounding the treated skin areas. The server 6 can inform the therapy planner 3 that the therapy plan has been automatically stopped because of the feedback of the patient 5 or for other circumstances, wherein the therapy planner 3 can be configured to allow the healthcare professional 4 to re-release the therapy plan. The therapy planner 3 also can be configured to allow the healthcare professional 4 to pause or cancel the therapy plan at any time.

Thus, the therapy planner 3 can be configured to allow the healthcare professional to a) input therapy parameters and to thereby, with the help of the servers, create therapy plans, b) edit therapy plans if necessary, c) monitor the feedback from the patients and d) release, pause or cancel therapy plans.

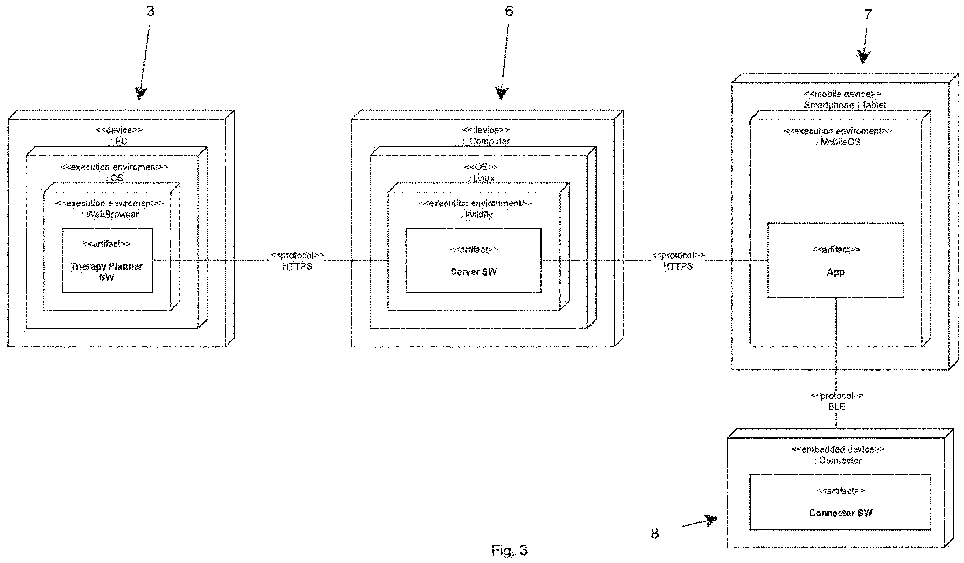

Fig. 3 schematically exemplarily illustrates that the therapy planner 3 might be an application running in a web browser on a personal computer (PC) having of course an operating system (OS). As also illustrated in Fig. 3, the server 6 can comprise server software (SW) in an operating system, for instance, Linux. As hardware, one or several computers can be provided for the server 6. If the therapy planer 3 is regarded as being a therapy manager front end, the server 6 could be regarded as being a backend, particularly a server-side backend. The server 6 can provide a RESTful API for providing the functionalities described herein. In particular, the server 6 does not only calculate the therapy plan and the control data, but it also manages data related to the light therapy. These data can include profiles of the patient, the therapy plans and data about the light sources, i.e. the therapy lamps, which might be used. For each light source at least one of the following data can be stored: the name of the respective light source, the UV band emitted by the respective light source and the light intensity emitted by the respective light source, wherein multiple light intensities might be stored for different distances between the skin and the respective light source. Thus, light source characteristics can be stored and provided by the server 6.

The patient data, which are stored on the server 6, can include a login name, personal data like the given name, the family name and the date of birth, data for reimbursement by a health insurance like a health insurance number of the patient and health insurance organization of the patient, the skin type of the patient, particularly a Fitzpatrick scale skin type, medication that may affect the photosensitivity of the skin, diagnoses of the patient and notes about the patient.

The therapy planner 3 is preferentially configured to allow the healthcare professional 4 to input the therapy parameters that are transferred to the server 6 which calculates the doses for the sessions based on the transferred therapy parameters. The therapy parameters can include one or several of the a) start date, b) the therapy device, c) a treatment area 1, d) an optional treatment area 2, e) an optional treatment area 3, f) a type of adjustment of the dose, g) a start dose, h) an action depending on a degree of redness or a degree of pain reported by the user 5 during a feedback procedure, i) a maximum dose, j) a total number of sessions, k) a number of sessions per week and I) a pause after how many weeks of inactivity.

The start date, i.e. element a) in the previous list, defines the date when a first session should be available for the patient. The therapy device, i.e. element b) in the previous list, defines the type of light source used for illuminating the skin. The treatment area 1, i.e. element c) in the previous list, defines the first treatment area to be irradiated. The optional treatment area 2, i.e. element d) in the previous list, defines the optional second treatment area to be irradiated and the treatment are 3, i.e. element e) in the previous list, defines the optional third treatment area to be irradiated.

Preferentially, the dose is increased from session to session, wherein the healthcare professional 4 can choose whether this increase should be as a fixed amount provided in, for instance, mJ/cm³ or as a percentage of the dose used during the previous session. In this embodiment percentage increase is a default. However, the healthcare professional 4 can also choose the type of adjustment, i.e. linear increase or percentage increase. This corresponds to element f) in the previous list.

The start dose, i.e. element g) in the previous list, defines the dose for the first session and is preferentially provided in mJ/cm². The action, i.e. element h) in the previous list, which depends on the degree of redness or degree of pain as provided by the patient 5 in his/her feedback, can include different actions depending on different degrees of redness or different degrees of pain.

For instance, during the feedback procedure the patient 5 can be asked how he/she would classify his/her specific degree of redness, wherein the patient 5 can choose between several predefined given degrees of redness. These different degrees can refer to i) no redness, ii) minimal redness and iii) strong redness. The patient device 7 can be configured to show to the patient 5 example images for the different degrees of redness, in order to allow the patient 5 to better assign his/her specific redness to one of the predefined degrees of redness. The actions, which can be assigned by the healthcare professional 4 to the different degrees of redness, can include increase of the dose, decrease of the dose, pausing the illumination until the healthcare professional 4 decides that the therapy can continue, asking the patient 5 to visit a healthcare professional, et cetera. In an embodiment, the therapy planner 3 is configured to allow the healthcare professional 4 to assign to the degree of redness "no redness" that the dose should be increased. The therapy planner 3 can be further configured to allow the healthcare professional 4 to assign to the degree of redness "minimal redness" that the dose should still be increased or that the dose should be reduced or that the therapy should be paused until the healthcare professional 4 indicates that the therapy can proceed. The therapy planner 3 also can be configured to allow the healthcare professional 4 to assign to the degree of redness "strong redness" that in this case the dose should still be increased or should be reduced or should be paused until the healthcare professional 4 has indicated that the therapy can proceed.

The maximum dose, i.e. element i) in the previous list, defines the dose after which the dose is not increased further and the total number of sessions, i.e. element j) in the previous list, defines the total number of sessions as it is clear. The number of sessions per week, i.e. element k) in the previous list, preferentially define the maximum number of sessions per week. The pause after how many weeks of inactivity, i.e. element I) in the previous list, defines the time in weeks after which the therapy is automatically paused, if the patient 5 does not do any of the planned sessions. The system is preferentially configured such that the therapy can start again only after the healthcare professional 4 has indicated via the therapy planner 3 that the therapy indeed can proceed.

The server 6 calculates the doses for the sessions based on the therapy parameters provided by the healthcare professional 4 via the therapy planner 3. In particular, the dose for the sessions is calculated by the server 6 based on the start dose and the maximum dose provided by the healthcare professional 4. Preferentially, also the type of adjustment as provided by the healthcare professional 4 via the therapy planner 3 is used for calculating the doses for the different sessions.

After a session has been carried out and if the patient 5 has provided feedback via the patient device 7 to the server 6, the server 6 can recalculate the doses for the immediately following session and preferentially for all remaining sessions based on the feedback. For recalculating the doses based on the feedback the servers can use the therapy parameters input by the healthcare professional 4 in a graphical user interface provided by the therapy planner 3. In particular, if the therapy parameters describe that the dose should be increased or decreased depending on a respective degree of redness, the server 6 can use this information for recalculating the doses for the remaining sessions. The server 6 can also use predefined knowledge regarding the slope of increase or of reduction of the dose depending on the respective degree of redness, wherein also these slopes can be chosen by the healthcare professional 4 via the therapy planner 3 or they can be predefined and stored in the system. If there is no feedback or the feedback indicates that there is no redness, in an embodiment the server 6 does not recalculate all doses.

The server 6 and the therapy planner 3 can be configured such that, after a dose for a next session and preferentially also for the previous sessions has been calculated by the server 6, they are shown to the healthcare professional 4 via the therapy planner 3, wherein the therapy planner 3 provides a graphical user interface allowing the healthcare professional 4 to modify the calculated doses. If the healthcare professional 4 has modified a dose for a certain session, the server 6 can adapt the doses of the other sessions by considering the dose manually modified by the healthcare professional 4. The recalculated doses can then again be shown to the healthcare professional 4 via the therapy planner 3, wherein the healthcare professional 4 then can either accept the recalculated doses or further modify one or several doses.

The sever 6 is configured to calculate the control data for a respective session based on the dose determined for the respective session. Preferentially, the server 6 uses for this calculation the parameters of the light source to be used for the sessions including the distance between the light source and the skin to be used during the sessions and the intensity of the light at this distance, wherein the intensity of the light can be given in, for instance, mW/cm². In a preferred embodiment the control data only include the resulting calculated illumination time which is sent from the server 6 to the patient device 7. In another embodiment, the control data can also include more information. The server can also use information about the body part to be irradiated for calculating the illumination time.

In an example the server 6 is configured to calculate the control data, i.e. preferentially the time period in which the light source is switched on, for an individual session based on the dose for the individual session as follows.

The dose may be calculated before or may have been manually set and may be given in mJ/cm² or in another unit like J/m². In the server 6 the distance between the light source and the body part is stored for the body part to be irradiated. This distance can be, for instance, part of light source characteristics and/or part of the patient data and/or part of the guidance data. In the server 6 it is also stored which light intensity is present at which distance for the light source to be used for the irradiation. This information can be stored as part of the light source characteristics. For instance, if in an example the light source is the light source dermaLight 500R-0, the light source characteristics can include that at a distance of 0 cm the light intensity is 12 mW/cm², at a distance of 3 cm the light intensity is 11 mW/cm² and at a distance of 30 cm the light intensity is 3 mW/cm². The server 6 can further be configured to calculate the control data, i.e. in this example the time period in which the light source should be switched on in this individual session, based on the dose and the intensity at the given distance. In particular, the server 6 can be configured to divide the dose by the light intensity at the given distance, in order to determine the time period especially in seconds during which the light source should be switched on.

Moreover, the server 6 preferentially is configured to calculate the dose especially based on the start dose provided by the healthcare professional 4 via the therapy planner 3 and an increase or decrease of the dose also set by the healthcare professional 4 via the therapy planner 3. The server 6 can be configured to calculate the doses for all sessions based on the assumption that there are no side effects when the therapy plan is created initially. The dose for the first session is set to be the start dose and the dose for each following session is calculated by the server 6 based on the dose of the respective previous session and the increase or decrease set by the healthcare professional 4 via the therapy planner 3 for the situation that no side effects, especially no redness, is present. The calculated dose is increased from session to session until a maximum dose has been reached, which also can be set by the healthcare professional 4 via the therapy planner 3.

The therapy planner 3 can be configured such that the dose increase and also the dose decrease can be set as a percentage of the previous dose for a previous session or as a fixed amount of dose increase between two subsequent sessions. For example, the therapy planner 3 can be configured to allow the healthcare professional 4 to set a dose increase of 15 %, if no redness can be seen, of 0 % if the skin is light red, of -10 % if the patient experiences medium redness and of -15 % for a dark red skin. The system preferentially is configured such that the execution of the therapy plan is paused until the patient confirms that the side effects are gone or until the patient has visited the healthcare professional 4.

As explained above, preferentially initially the server 6 calculates the doses for all sessions based on the assumption that no side effects will occur. Thus, if a side effect occurs, the doses for all following sessions preferentially are recalculated. In particular, the server 6 recalculates the dose for the next session based on the increase or decrease value set by the healthcare professional 4 via the therapy planner 3 for the level of redness indicated by the patient after the previous session. After the dose has been calculated for the next session, the dose for all remaining sessions is recalculated based on the dose for the next session and based on the assumption that no redness will occur in the remaining sessions.The server 6 can be configured to remind the patient device 7 and thereby the patient 5 of an available session and that feedback is due. For this purpose, the server 6 can be configured to send push notifications to the patient device 7. Preferentially, besides the push notifications, all communication between the server 6 and the therapy planner 3 or the patient device 7 is initiated by the therapy planner 3 or the patient device 7. The server 6 preferentially never sends data to the therapy planner 3 or the patient device 7 without a previous request, except for the above-mentioned push notifications that might be sent from the server 6 to the patient device 7 without a previous request from the patient device 7 to the server 6. As explained above, the therapy planner 3 and the patient device 7 preferentially communicate with the server 6 via the Internet, wherein preferentially the HTTP protocol is used.

Referring again to Fig. 3, the patient device 7 preferentially is a smartphone or a tablet computer on which a software, i.e. an app, is executed in the environment provided by the operating system of the smartphone or tablet computer. The patient device 7, i.e., for instance, the smartphone or tablet computer with the corresponding app, is used by the patient 5 to retrieve the control data and other instructions like guidance data for the current session and to provide feedback about the outcome of the treatment, particularly about unwanted side effects.

The patient device 7 with the app, which could be regarded as being a therapy app, can be used by the patient 5 to conduct the therapy sessions. The patient device 7, in particular, the app running on the patient device 7, can provide at least one of the following functionalities: a) receiving the control data for the current session from the server 6, wherein, if no current session is available, the server 6 informs the patient device 7 accordingly and the patient device 7 then informs the patient 5 about the time until the next session is available as defined by the therapy plan, b) providing information about the current state of the therapy, and c) the option to allow the patient 5 to signal to the healthcare professional 4 that symptoms of side effects are gone. Further functionalities, which can be provided by the patient device 7, refer to d) guiding the patient 5 through the respective session based on guidance data received from the server 6, wherein the guiding can include providing safety checks and guidance for the setup of the light source 2 that, as mentioned above, might also be regarded as being a therapy lamp. The guidance can also include that the patient 5 is advised to use certain skin care products to avoid dry skin, after a session has been completed. A further functionality, that can be provided by the patient device 7, refers to e) allowing the patient 5 to provide feedback a predetermined time after the end of the last session, in order to inform the healthcare professional 4 about any side effects like redness of the treated skin or surrounding skin, pain in the treated area, et cetera. The predetermined time after the end of the previous session can be, for instance, 22 hours. The patient device 7 and also the therapy planner 3 provide user interfaces, particularly graphical user interfaces, which allow the patient 5 and the healthcare professional 4, respectively, to interact with the patient device 7 and the therapy planner 3, respectively.

The connector 8, which is also illustrated in Fig. 3, comprises connector software and is configured to provide the functions of the connector 8 described herein. The connector 8 receives the control data for the current session from the patient device 7, i.e. in this embodiment from the app running on the smartphone or tablet computer, and controls the light source 2 in accordance with the control data. For instance, the control data can indicate the illumination time for the respective session and the connector 8 can be configured to switch on the light source 2 for exactly this illumination time, i.e. exactly for this amount of time. The connector can comprise a Bluetooth module or another communication module for communicating with the patient device 7, a timer and electronic components to control the current of the therapy lamp, i.e. in order to switch on the therapy lamp exactly for the time period defined by the control data. In an embodiment the connector 8 is directly connected to the voltage and/or current input of the light source 2. Thus, the light source 2 can be a known light source which normally would be controlled by a normal known controller that is connected to the voltage and/or current input of the known light source. This normal known controller can be replaced by the connector 8 such that the connector 8 controls the known light source by providing voltage and/or current to the known light source in accordance with the received control data.

The light source 2, i.e. the therapy lamp, is connected to the separate connector 8 in this embodiment. In another embodiment, the connector can also be integrated in the light source, i.e. the light source can itself already be compatible with the further components of the system. Thus, the integrated connector might also be regarded as being a control module that is configured to generate a data connection between the patient device and the light source and to control the light source in accordance with the received control data. Moreover, the patient device 7 then can be configured to transfer the control data for the current session especially wirelessly to the light source 2 using a wireless protocol like Bluetooth (BLE).

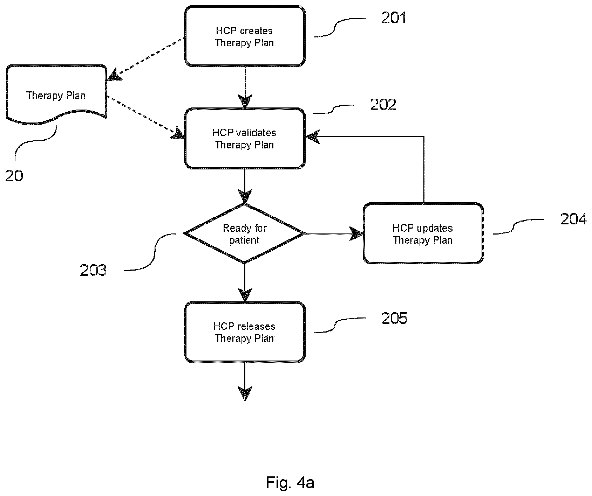

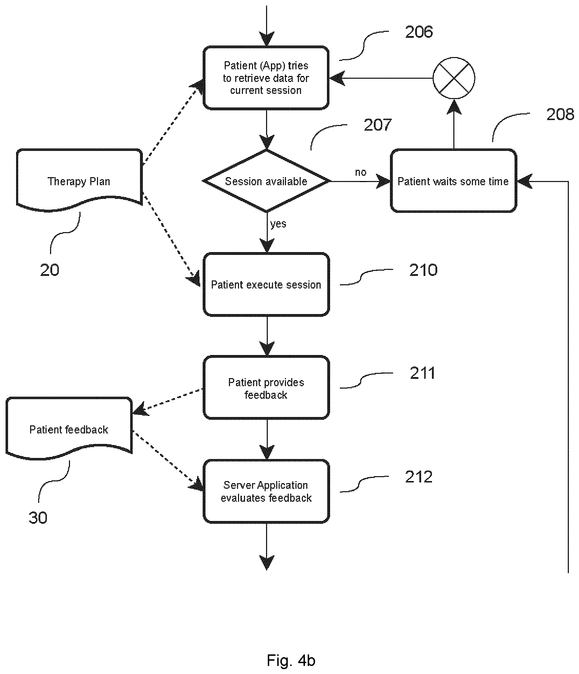

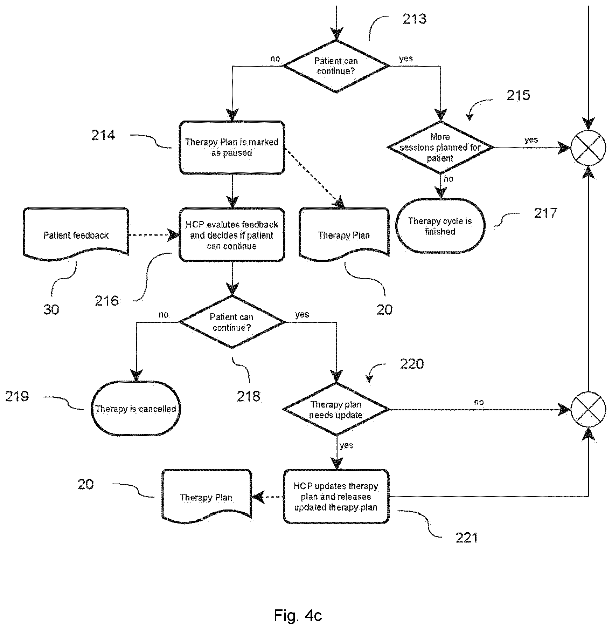

Figs. 4a, 4b, 4c exemplarily illustrate further details of the method for controlling the light source 2.

In step 201 the therapy planner 3 allows the healthcare professional 4 to input therapy parameters that are transferred to the server 6 that creates a therapy plan 20, wherein in step 202 the therapy planner 3 further allows the healthcare professional 4 to validate and/or check the therapy plan 20. If, in step 203, the healthcare professional 4 indicates via a corresponding user interface provided by the therapy planner 3 that the therapy plan 20 is ready, the therapy plan 20 is released in step 205. Otherwise, in step 204 the therapy planner 3 requests the healthcare professional 4 to update the therapy plan directly or to update the therapy parameters which then are used by the server 6 to update the therapy plan. Releasing the therapy plan makes the therapy plan available for the patient 5, wherein, otherwise, as mentioned before, the healthcare professional 4 can customize the therapy plan 20 until the healthcare professional 4 is satisfied with the therapy plan 20.

The therapy parameters can comprise the following parameters: a) the type of light source, i.e. the type of therapy lamp, to be used, b) the body part to be irradiated, c) the distance between the light source and the body part, i.e. between the light source and the skin of the body part, to be used during the treatment, d) whether the light source should be used with or without an accessory that helps to keep the distance, i.e. with or without a distance holder, e) the start dose preferentially in mJ/cm² that is the dose to be used in the first session, f) the maximum dose preferentially in mJ/cm² to be used during the therapy, g) the number of sessions of the therapy plan, h) the start date, i.e. the date of the first session, i) a set of dose adjustments for different redness levels and j) the maximum number of sessions in a predefined period that might be a seven-day period.

The set of dose adjustments for different redness levels can include, for each redness level, the value of increasing or decreasing the dose based on the dose of the last session, and, whether the dose should be reset to the start dose if the patient develops a redness of a specified level. The therapy parameters also can include information defining whether the patient can continue or should pause if a redness of a specified level is observed. The therapy plan of course is assigned to a specific patient and therefore includes an indication to the patient like the patient's name and the patient's date of birth, and the therapy plan also can include information about a previous diagnosis of the patient. Thus, the therapy plan also can comprise some patient data.

For different light sources, corresponding light source characteristics can be stored in the server 6. Thus, for instance, a UV band emitted by the respective light source and the light intensities emitted at different distances by the light source can be provided, for instance, in mW/cm². As a further light source characteristic, in the server 6 it can also be stored which configuration of the light source is to be used for a respective treatment area. In particular, for each treatment area the following data can be stored: a) whether the light source is suitable or unsuitable for the respective treatment area and b) whether the light source needs to be used with an accessory or without an accessory for treating the respective treatment area. If an accessory is needed, also the type of accessory can be stored in the server 6. The accessory helps to keep especially short distances to the light source. These configuration data can also include the distance that should be used during a therapy session.

Referring to Fig. 4b, the patient device 7 can request the data, in particular, the control data, for a current session in step 206. If the server 6 responds that no session is currently available, the patient waits some time (step 208) and uses the patient device 7 later for requesting data of a current session. If a current session is available, the current session is provided from the server 6 to the patient device 7 and, in step 210, the patient device 7 uses the connector 8 for controlling the light source 2 in accordance with the control data received from the server 6. Thus, in step 210, the patient device 7 executes the current session of the therapy plan. The server 6 will provide the patient device 7 only with the data for the current session, in order to avoid errors like carrying out a wrong session. Moreover, preferentially the control data for a next session are determined by the server 6 only, after the patient device 7 has requested the next session. In particular, the illumination time, i.e. the time period during which the skin should be illuminated, for the next session is determined by the server 6 only, after the patient device 7 has requested the next session, and not before. This reduces computational efforts, because control data are not calculated unnecessarily in advance.

After a session has been completed, the patient device 7 asks the patient for feedback. Preferentially, the patient device 7 asks the patient for feedback only after a certain time period which might be 22 hours. The feedback can be, for instance, whether the treated skin area or the skin surrounding the treated skin area is red, wherein whether the treated area or the skin surrounding the treated area is relevant for the feedback can depend on the specific disease treated. Thus, the patient device 7 can ask for a feedback regarding the treated area and/or the surrounding area based on the disease treated. The server 6 or the patient device 7 can comprise corresponding rules defining for which disease for which feedback is asked. The feedback, for which the patient is asked, can also include whether the patient has any pain in the treated area or the surrounding skin. These feedback data can also include optional comments, wherein, if pain is reported, a comment by the patient might be mandatory. The patient device 7 also adds date and time of the feedback to these data.

In step 211 the feedback data 30 are transmitted to the server 6, wherein, in step 212, the received feedback data 30 are evaluated by the server 6. In particular, based on the dose adjustments that might be included in the therapy parameters based on specific levels of redness, the server 6 can evaluate whether the patient can continue with the therapy without amending the therapy plan or whether the therapy plan needs, for instance, an update. This decision is made by the server 6 in step 213. If the server 6 decides that the patient can continue, in step 215 the server 6 checks whether there are more sessions planned for the patient as defined by the therapy plan. If this is also the case, the method continues with step 208. If the server decides in step 213 that the patient cannot continue with the therapy plan, in this embodiment the therapy plan is paused in step 214 and the patient device 7 will not receive control data of any new sessions until the therapy plan has been released again by the therapy planner 3 in response to a corresponding input made by the healthcare professional 4.

In step 216 the feedback data are transmitted from the server 6 to the therapy planner 3, wherein the therapy planner 3 provides a user interface that allows the healthcare professional 4 to evaluate the feedback data and to decide if the patient 5 can continue with the therapy. If in step 218 it is decided that the patient 5 can continue with the therapy, in step 220 the therapy planner 3 asks the healthcare professional 4 whether the therapy plan needs an update. If this is not the case, this information is provided from the therapy planner 3 to the server 6 and the method continues with step 208. If the therapy plan needs an update, the therapy planner 3 provides a user interface to the healthcare professional 4, which allows the healthcare professional 4 to update the therapy plan directly or to update the therapy parameters which then are transferred to the server 6 which updates the therapy plan and to release the updated therapy plan in step 221. The method then continues with step 206. If in step 218 it is decided that the patient 4 cannot continue, the method is stopped in step 219.

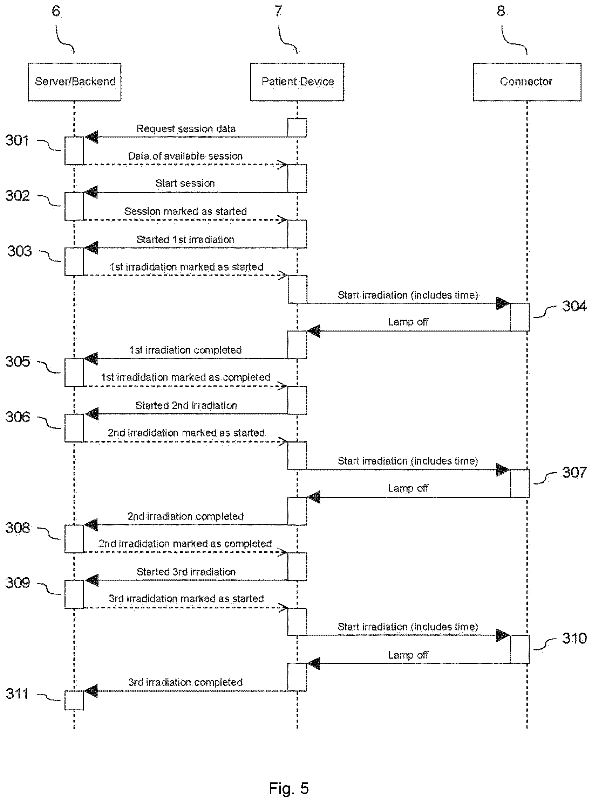

Fig. 5 shows an UML Sequence Diagram for illustrating an execution of a session, wherein in an embodiment a session can comprise up to three different irradiations for different treatment areas, wherein the treatment areas and also the irradiations to be applied to the treatment areas are defined by the therapy plan.

In step 301 the patient device 7 asks the server 6 whether a current session is available. If a current session is available, as it is assumed in Fig. 5, the server 6 determines the control data, particularly the illumination time, based on the therapy parameters and transmits the data, especially the control data, required by the patient device 7 for executing the current session from the server 6 to the patient device 7. The data transferred from the server 6 to the patient device 7 in step 301 include, for each irradiation to be carried out during the current session, the treatment area to be irradiated, the distance between the light source and the skin to be used, whether an accessory like a distance holder should be used for the irradiation, and the irradiation time, i.e. the time between switching the light source on and off.

In an embodiment, the server 6 stores the radiation doses defined in the therapy plan in mJ/cm² for each session. In this case, when a current session is required by the patient device 7 in step 301, the server 6 calculates the duration of applying the light for the respective irradiation based on the distance to use. The distance to use can be a fixed value for the respective light source, wherein in this case for this calculation just this fixed distance can be used. The distance can also be defined by the therapy parameters, wherein in this case the distance defined by the therapy parameters can be used for this calculation.

After the patient has started the current session, the patient device 7 informs the server 6 accordingly and the server 6 marks the session as started in its database in step 302.

The patient device 7 then guides the patient through the setup of the light source and the preparation for the current session, wherein this can include using the right distance between the skin and the light source, that the areas to be irradiated are not covered by clothes or jewelry and that the patient wears safety glasses. This setup preparation procedure can be repeated for each irradiation of the current session. Corresponding guidance data, which define this setup and preparation procedure, can be provided for the current session from the server 6 to the patient device 7.

In step 303 the patient device 7 informs the server 6 that the first irradiation has been started and the server 6 marks the first irradiation in its database as being started. In step 304 the patient device 7 requests the connector 8 to switch the light source 2 on and the patient device 7 also transmits the irradiation time for the first irradiation of the current session to the connector 8.

After a short delay, the connector 8 switches the light source 2 on. The short delay of, for instance, 5 seconds, is used to allow the patient to put down the patient device 7 and (re)assume the correct position, i.e. the position defined during the setup and preparation procedure. After the time duration for the respective treatment of the current session, the connector 8 switches the light source 2 off and informs the patient device 7 that the light source 2 has been switched off (still in step 304).

The patient device 7 transmits the information that the first irradiation has been finished to the server 6 which marks the first irradiation as completed in its database. If there are no further irradiations for the current sessions, the server 6 also marks the current session as completed. In steps 306 to 311, the steps 303 to 305 explained above for the first irradiation are repeated for a second irradiation and a third irradiation of the same current session.

Although not shown in Fig. 5, the connector 8 can also be configured to report the activation of the light source to the patient device 7 and only thereafter the patient device 7 informs the server 6 about the start of the respective irradiation. Moreover, it is also possible that the patient device 7 informs the server 6 about the start of the respective irradiation, only after the patient device 7 has requested the connector 8 to switch the light source 2 on.

After the current session has been completed, in a preferred embodiment the patient device 7 has to wait 22 hours or another predefined time period, before it can ask the patient for feedback regarding the just completed session. Using 22 hours or another time, which allows the patient 5 to complete the feedback provision before 24 hours after the end of the previous session have lapsed, is advantageous, because it avoids a slow drift of the session times during the entire therapy cycle.

The connector 8 is preferentially configured to not have any communication with the patient device 7 while the light source 2 is switched on. The connector 8 therefore autonomously switches the light source 2 off, after the light source 2 was on for the time defined by the control data, without relying on any communication with the patient device 7. This ensures that the light source 2 is switched off at the right time independently of any generally possible communication problem between the connector 8 and the patient device 7 and independently of any malfunction of the patient device 7. This increases the safeness of the light treatment without a healthcare professional being present for directly observing the light treatment. The connector 8 can be configured, as explained above, to report the activation of the light source 2 to the patient device 7, when the light source 2 has been switched on, wherein thereafter preferentially there is not any communication between the connector 8 and the patient device 7. In another embodiment, also during the time period, in which the light source 2 is switched on, there is some kind of communication between the connector 8 and the patient device 7, wherein in this case the connector 8 is still configured to autonomously switch the light source 2 off, after the end of the illumination period has been reached as indicated by the control data, without being influenced by the communication between the connector 8 and the patient device 7.

A session may be cancelled due to several reasons. Until the cancellation, the sequence can be the same as described above with reference to Fig. 5, i.e. it can be the same as when executing an irradiation. The possible reasons for cancelling an irradiation of a current session are, for instance, an emergency-off button on the connector 8 is pressed, the connector 8 detects an error or the patient device 7 detects an error. If, for instance, the emergency-off button is pressed or the connector 8 detects an error during an irradiation of the current session, in step 401 this is reported from the connector 8 to the patient device 7, wherein then this is further reported from the patient device 7 to the server 6 in step 402. The server 6 then marks the irradiation and also the entire current session as cancelled. If available, the patient device 7 can also send additional data of the cancellation like error codes to the server 6. The patient device 7 can also be configured such that, if the cancellation has been caused by the patient 5 pressing the emergency-off button, the patient device 6 asks the patient 5 to provide a reason why the patient 5 pressed the emergency-off button. Also this information then will be transmitted from the patient device 7 to the server 6.

The cancellation message sent from the patient device 7 to the server 6 will include the cause of cancellation like the error code or any information provided by the patient regarding the cancellation. This information then can also be transmitted from the server 6 to the therapy planner 3. The therapy planner 3 can be configured to ask the healthcare professional 4 whether the therapy plan can be re-released. In an embodiment, the therapy planner 3 can be configured to allow the healthcare professional 4 to release the therapy plan, before the patient 5 provided feedback for the cancelled session. In an embodiment, if the patient 5 did not report any problems for the cancelled session, the therapy plan may continue normally, without further intervention from the healthcare professional 4. The next session then will use the same dose as the cancelled session and the control data for following sessions will be recalculated if necessary.

In a preferred embodiment, also after a cancellation, the patient device 7 is configured to request feedback about possible skin reactions a predefined time after the session has been cancelled, wherein this predefined time might be 22 hours. The patient will not be able to continue the therapy before a healthcare professional has re-released the therapy plan.

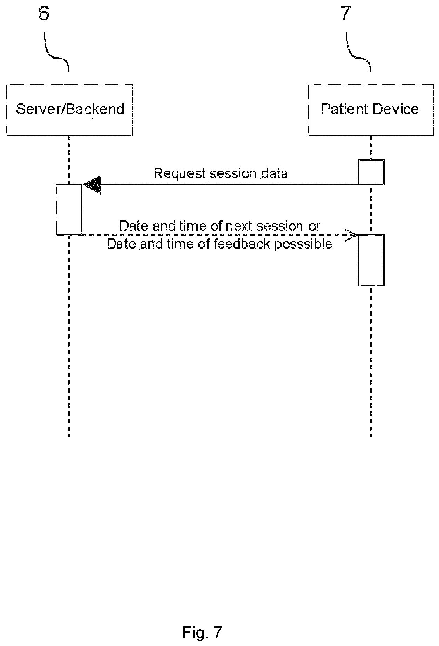

After a session has been completed, the patient has to wait until feedback is possible and until a next session is available. In a preferred embodiment, in both cases the patient device 7 requests the data, in particular, the control data, for the next session from the server 6. However, instead of the data for the next session, the server 6 will either respond with a message that the patient has to wait until feedback is possible or until the next session becomes available. These messages contain the date and time when feedback is possible or when the next session becomes available so that the patient device 7 can display, for instance, the time until feedback is possible or until the next session becomes available. This is illustrated in Fig. 7.

Preferentially a predefined time after a session, for instance, 22 hours after a session, regardless whether the session was completed or cancelled, the patient must provide feedback about skin reactions in or around the treated areas. Whether the required feedback is about the treated skin or the skin around the treated area depends on the diagnosis or indication treated with the current therapy plan of the patient.

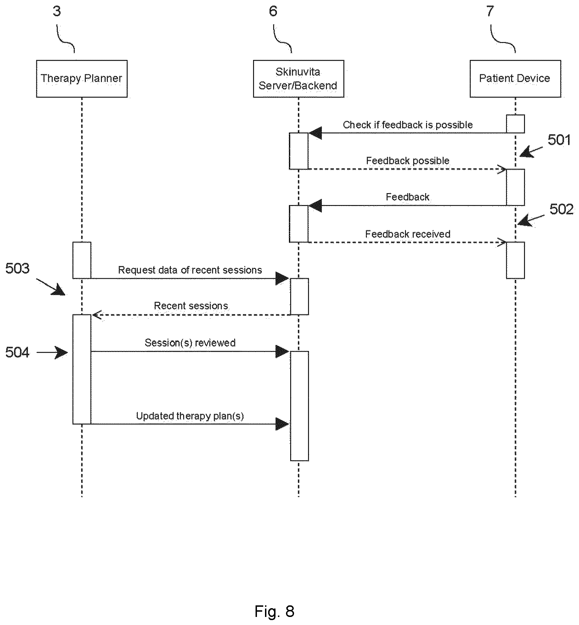

As illustrated in Fig. 8, the sequence of providing feedback can start with the patient device 7 asking the server 6 if feedback is possible in step 501. If feedback is possible, the server 6 informs the patient device 7 accordingly still in step 501. The server 6 thereby sends to the patient device 7 information about the treated skin areas so that the patient device 7 can request feedback focused on the treatment areas. The patient device then asks the patient for feedback regarding, for instance, the level of redness of the treated skin area or of the skin around the treated area or whether the patient experienced any pain in the treated area or around the treated area. The patient device 7 also can ask the patient to take a photo of the treated skin area or the skin around the treated area as a feedback. The patient device 7 also can ask the patient for a feedback comment. For instance, if the patient reports pain, the patient device 7 can be configured to ask the patient for details regarding the pain.

In step 502 the feedback data is sent from the patient device 7 to the server 6. The server 6 preferentially is configured to initiate actions based on the feedback and based on parameters of the therapy plan. For instance, an action can be that the therapy for the patient 5 should be stopped. Even if the feedback does not indicate any problem, as an action the server 6 can mark the respective session as to be reviewed. It can also be defined that, after a certain number of sessions has been completed, a reviewing procedure needs to be started, even if the feedback data did not include any problem. The reviewing procedure includes allowing the healthcare professional 4 to review the previous sessions by using the therapy planner 3. The server 6 can also be configured to update the radiation doses for all remaining sessions based on the therapy parameters and the feedback of the patient.

The therapy planner 3 can also request the feedback data for the sessions which have been completed already in step 503. For each reviewed session, the therapy planner 3 can send the information that the respective session has been reviewed to the servers, wherein then the server 6 can mark the session as being reviewed in its database. The therapy planner 3 can be configured to allow the therapy plans of the patients to be modified by the healthcare professional 4, wherein, after the healthcare professional 4 has made corresponding changes, the therapy planner 3 sends the changes to the server 6 that stores the changes in its database and updates the parameters for the remaining sessions of the therapy cycle.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

The control of the system for controlling the light source in accordance with the method for controlling the light source can be implemented as program code means of a computer and/or as dedicated hardware. Also the control of the therapy planner for causing the therapy planner to carry out the above described steps or the control of the server to carry out the above described steps can be implemented as program code means of a computer program and/or as dedicated hardware. Moreover, the control of the patient device in accordance with the above described steps and the control of the connector in accordance with the above described steps can be implemented as program code means of a computer and/or as dedicated hardware.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

The invention relates to a system for controlling a light source. A therapy planner allows a user to input therapy parameters usable for generating a light therapy plan for a patient, which defines several sessions of a light therapy to be applied to the skin of the patient and comprises dose data indicative of the radiation dose to be applied during a respective session and time data indicative of the time at which the respective session is to be carried out. A server calculates control data for the several sessions based on the dose data of the light therapy plan, wherein the control data define an operation of the light source during the respective session. A patient device receives the control data from the server and transfers the control data to a connector which controls the light source in accordance with the received control data.

## Claims

1. A system (1) for controlling a light source (2), the system (1) comprising:
- a therapy planner (3) configured to allow a user (4) to input therapy parameters that are usable for generating a light therapy plan for a patient (5), wherein the light therapy plan defines several sessions of a light therapy to be applied to the skin of the patient (5) and comprises dose data indicative of the radiation dose to be applied during a respective session and time data indicative of the time at which the respective session is to be carried out,
- a server (6) configured to calculate the light therapy plan based on the input therapy parameters and to calculate control data for the several sessions based on the dose data of the light therapy plan, wherein the control data define an operation of the light source (2) during the respective session,
- a patient device (7) configured to receive the control data from the server (6) and to transfer the control data to a connector (8),
- the connector (8) configured to connect the patient device (7) with the light source (2), wherein the connector (8) is configured to control the light source (2) in accordance with the received control data.

2. The system (1) as defined by claim 1, wherein the server (6) comprises light source data indicative of light source characteristics, wherein the server (6) is configured to calculate the control data further based on the light source characteristics, wherein the light source characteristics particularly include information about at least one of:
- the spectral distribution, particular an ultraviolet band, of the light,
- the light intensity providable by the light at at least one distance between the light source (2) and the skin of the patient (5).

3. The system (1) as defined by any of the preceding claims, wherein the server (6) comprises patient data, wherein the server (6) is configured to calculate the light therapy plan further based on the patient data, wherein the patient data particularly include information about at least one of:
- the skin type of the patient (5),
- medication of the patient (5),
- diagnoses of the patient (5),
- feedback data from the patient (5) about effects of the light therapy.

4. The system (1) as defined by any of the preceding claims, wherein the control data includes a time period of applying the radiation during the respective session.

5. The system (1) as defined by any of the preceding claims, wherein the server (6) further is configured to determine guidance data for guiding the patient (5) before and/or during a session and to provide the guidance data to the patient device (7), wherein the guidance data particularly include at least one of:
- a distance between the light source (2) and the skin to be used during the respective session,
- guidance regarding clothing of the patient (5) during the respective session,
- whether to wear safety glasses.

6. The system (1) as defined by any of the preceding claims, wherein a bi-directional communication a) between the server (6) and the therapy planner (3) and b) between the server (6) and the patient device (7) is initiated only by the therapy planner (3) and the patient device (7), respectively, and not by the server (6).

7. The system (1) as defined by any of the preceding claims, wherein the server (6) is configured to:
- check whether a next session is available based on a current time and the time data of the different sessions, if the patient device (7) requests control data for the next session,
- provide the control data of the next session to the patient device (7), if the check revealed that a next session is available.

8. The system (1) as defined by any of the preceding claims, wherein the server (6) is configured to inform the patient device (7) when a next session is to be carried out based on the time data of the sessions as defined by the therapy plan.

9. The system (1) as defined by any of the preceding claims, wherein the patient device (7) is configured to ask the server (6) whether it is possible to provide feedback and to allow the patient (5) to input feedback, if the server (6) has informed the patient device (7) that feedback is possible.

10. The system (1) as defined by any of the preceding claims, wherein the patient device (7) includes a camera and is configured to:
- ask the patient (5) to make a photo of the treated skin area,
- send the photo to the server (6) as feedback,
wherein particularly the server (6) is configured to analyze the photo, for example by artificial intelligence, and to adapt the therapy plan based on the result of the analyses.

11. A connector (8) for a system for controlling a light source (2) as defined by any of claims 1 to 10, wherein the connector (8) is configured to connect the patient device (7) with the light source (2), wherein the connector (8) is configured to control the light source (2) in accordance with the received control data.

12. A method for controlling a light source (2), the method comprising:
- allowing a user (4) to input therapy parameters that are usable for generating a light therapy plan for a patient (5) by a therapy planner (3), wherein the light therapy plan defines several sessions of a light therapy to be applied to the skin of the patient (5) and comprises dose data indicative of the radiation dose to be applied during a respective session and time data indicative of the time at which the respective session is to be carried out,
- calculating the light therapy plan based on the input therapy parameters and calculating control data for the several sessions based on the dose data of the light therapy plan by a server (6), wherein the control data define an operation of the light source (2) during the respective session,
- receiving the control data from the server (6) and transferring the control data to a connector (8) by a patient device (7),
- controlling the light source (2) in accordance with the received control data by the connector (8).

13. A method to be carried out by a connector (8) of a system for controlling a light source (2) as defined by any of claims 1 to 10, wherein the connector (8) controls the light source (2) in accordance with the received control data.

14. A computer program for controlling a light source, the computer program comprising program code means for causing the system for controlling the light source as defined by any of claims 1 to 10 to carry out the steps of claim 12.

15. A computer program for controlling a connector (8) of a system (1) for controlling a light source (2) as defined by any of claims 1 to 10, the computer program comprising program code means for causing the connector (8) to carry out the method as defined by claim 13.
